# EUROPEAN PATENT SPECIFICATION

(11) **EP 1 646 634 B1**
(45) Date of publication and mention of the grant of the patent: **12.11.2008**
(21) Application number: 04740750.7
(22) Date of filing: 07.07.2004
(51) Int. Cl.: C07D 498/18, A61K 31/436, A61P 19/00

(54) **USE OF RAPAMYCIN AND RAPAMYCIN DERIVATIVES FOR THE TREATMENT OF BONE LOSS**
VERWENDUNG VON RAPAMYCIN UND DESSEN DERIVATEN ZUR BEHANDLUNG VON KNOCHENVERLUST
UTILISATION DE RAPAMYCINE ET DE DERIVES DE RAPAMYCINE POUR TRAITER LES PERTES DE MASSE OSSEUSE

(30) Priority: 08.07.2003 GB 0315965; 08.07.2003 GB 0315963
(43) Date of publication of application: 19.04.2006
(73) Proprietor: NOVARTIS AG, 4056 Basel (CH)
(72) Inventor: KNEISSEL, Michaela, 4057-Basel (CH); SUSA SPRING, Mira, CH-4055 Basel (CH)
(74) Representative: Roth, Peter Richard
(86) International application number: PCT/EP2004/007437
(87) International publication number: WO 2005/005434

(56) References cited:
- US-A- 5 258 389
- US-A- 5 527 907
- SHUI C ET AL: "The immunosuppressant rapamycin, alone or with transforming growth factor-beta, enhances osteoclast differentiation of RAW264.7 monocyte-macrophage cells in the presence of RANK-ligand." CALCIFIED TISSUE INTERNATIONAL, vol. 71, no. 5, November 2002 (2002-11), pages 437-446, XP002299513 ISSN: 0171-967X
- ROMERO DAVID F ET AL: "Rapamycin: A bone sparing immunosuppressant?" JOURNAL OF BONE AND MINERAL RESEARCH, vol. 10, no. 5, 1995, pages 760-768, XP008036532 ISSN: 0884-0431
- VAN ETTEN EVELYNE ET AL: "Analogs of 1,25-dihydroxyvitamin D3 as dose-reducing agents for classical immunosuppressants" TRANSPLANTATION (BALTIMORE), vol. 69, no. 9, 15 May 2000 (2000-05-15), pages 1932-1942, XP008036525 ISSN: 0041-1337
- CAI J, JAMIESON C, MOIR J, RANKOVIC Z: "Cathepsin K inhibitors" EXPERT OPINION ON THERAPEUTIC PATENTS, vol. 15, no. 1, 2005, pages 33-48,
- EXPERT OPINION ON THERAPEUTIC PATENTS, vol. 13, no. 1, 2003, pages 59-66, Selective androgen receptor modulators

## Description

The present invention relates to a new use of rapamycin derivatives.

Rapamycin is an immunosuppressive lactam macrolide that is produced by Streptomyces hygroscopicus.

A rapamycin derivative is a substituted rapamycin e.g. a 40-O-substituted rapamycin e.g. as described in US 5 258 389, WO 94/09010, WO 92/05179, US 5 118 677, US 5 118 678, US 5 100 883, US 5 151 413, US 5 120 842, WO 93/11130, WO 94/02136, WO 94/02485 and WO 95/14023, a 16-O-substituted rapamycin e.g. as disclosed in WO 94/02136, WO 95/16691 and WO 96/41807, or a 32-hydrogenated rapamycin e.g. as described in WO 96/41807 and US 5 256 790.

Preferred rapamycin derivatives are compounds of formula I 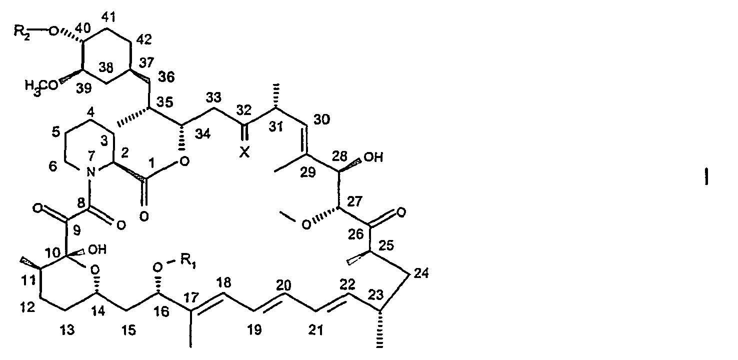 wherein
R₁ is CH₃ or C₃₋₆alkynyl,
R₂ is H or -CH₂-CH₂-OH, 3-hydroxy-2-(hydroxymethyl)-2-methyl-propanoyl or tetrazolyl, and X is =O, (H,H) or (H,OH)
provided that R₂ is other than H when X is =O and R₁ is CH₃,
or a physiologically hydrolysable ether thereof when R₂ is -CH₂-CH₂-OH.

Particularly preferred rapamycin derivatives of formula I are 40-O-(2-hydroxyethyl)-rapamycin (Compound A hereinafter), 40-[3-hydroxy-2-(hydroxymethyl)-2-methylpropanoate]-rapamycin (also called CCl779) 40-epi-(tetrazolyl)-rapamycin (also called ABT578), 32-deoxorapamycin, 16-pent-2-ynyloxy-32(S)-dihydro rapamycin, or TAFA-93. Even more preferred is Compound A.

Rapamycin derivatives also include so-called rapalogs, e.g. as disclosed in WO 98/02441 and WO 01/14387, e.g. AP23573, AP23464, or AP23841.

Rapamycin and derivatives thereof have, on the basis of observed activity, e.g. binding to macrophilin-12 (also known as FK-506 binding protein or FKBP-12), e.g. as described in WO 94/09010, WO 95/16691 or WO 96/41807, been found to be useful e.g. as immuno-suppressant, e.g. in the treatment of acute allograft rejection.

It has now been found that rapamycin derivatives are useful for the treatment of abnormally increased bone turnover or resorption.

In accordance with the particular findings of the present invention, there is provided:
1. Rapamycin derivatives for use in treating increased bone turnover or resorption in a subject in need thereof, comprising administering to said subject a therapeutically effective amount of a rapamycin derivative.
   In particular, there is provided:
   1.1 Rapamycin derivatives for use in treating osteoporosis, e.g. postmenopausal osteoporosis, postmenopausal bone loss; male osteoporosis; corticosteroid-induced osteoporosis, in a subject in need thereof, comprising administering to said subject a therapeutically effective amount of a rapamycin derivative.
   1.2 Rapamycin derivatives for use in treating bone loss secondary to or due to medication, e.g. diphenyl-hydantoin, thyroid hormone replacement therapy; in a subject in need thereof, comprising administering to said subject a therapeutically effective amount of a rapamycin derivative.
   1.3 Rapamycin derivatives for use in treating bone loss associated with immobilisation and space flight; in a subject in need thereof, comprising administering to said subject a therapeutically effective amount of a rapamycin derivative.
   1.4 Rapamycin derivatives for use in treating bone loss associated with rheumatoid arthritis, osteopenia, osteogenesis imperfecta, hyperthyroidism, anorexia nervosa, organ transplantation, joint prosthesis loosening, in a subject in need thereof, comprising administering to said subject a therapeutically effective amount of a rapamycin derivative.
   1.5 Rapamycin derivatives for use in treating periarticular bone erosions in rheumatoid arthritis, in a subject in need thereof, comprising administering to said subject a therapeutically effective amount of a rapamycin derivative.
   1.6 Rapamycin derivatives for use in treating osteoarthritis, e.g. subchondral osteosclerosis, subchondral bone cysts, osteophyte formation, and of osteoarthritic pain, e.g. by reduction in intra-osseous pressure, in a subject in need thereof, comprising administering to said subject a therapeutically effective amount of a rapamycin derivative.
   1.7 Rapamycin derivatives for use in treating hypercalcemia, e.g. tumour-induced hypercalcemia, e.g. resulting from excessive bone resorption secondary to hyperparathyroidism, thyrotoxicosis, sarcoidosis or hypervitaminosis D, in a subject in need thereof, comprising administering to said subject a therapeutically effective amount of a rapamycin derivative.
   1.8 Rapamycin derivatives for use in treating bone cancer and bone metastases, in a subject in need thereof, comprising administering to said subject a therapeutically effective amount of a rapamycin derivative; in particular a method for treating bone cancer and bone metastases induced by a primary tumour, e.g. breast or prostate cancer.

   In the present description the terms "treatment" or "treat" refer to both prophylactic or preventive treatment as well as curative or disease modifying treatment, including treatment of patients at risk of contracting the disease or suspected to have contracted the disease as well as patients who are ill or have been diagnosed as suffering from a disease or medical condition.
   In a series of further specific or alternative embodiments, the present invention also provides:
2. Rapamycin derivative for use in any method as defined under 1, in particular under 1.1 to 1.8 above.
3. Rapamycin derivative for use in the preparation of a pharmaceutical composition for use in any method as defined under 1, in particular under 1.1 to 1.8 above.
4. A pharmaceutical composition for use in any method as defined under 1, in particular under 1.1 to 1.8 above, comprising rapamycin derivative together with one or more pharmaceutically acceptable diluents or carriers therefore.
   Rapamycin derivative may be administered as the sole drug or in combination with a second drug. Suitable drugs for combination include a bone resorption inhibitor, e.g. as in osteoporosis therapy, in particular a calcitonin or an analogue or derivative thereof, e.g. salmon, eel or human calcitonin; a steroid hormone, e.g. an estrogen, a partial estrogen agonist or estrogen-gestagen combination; a selective estrogen receptor modulator (SERM) e.g. raloxifene, lasofoxifene, TSE-424, FC1271; tibolone (Livial ®); vitamin D or an analogue thereof; Parathyroid Hormone (PTH), a PTH fragment or a PTH derivative e.g. PTH (1-84), PTH (1-34), PTH (1-36), PTH (1-38), PTH (1-31)NH2 or PTS 893; a bisphosphonate e.g. alendronate, zoledronic acid, ibandronate; a cathepsin K inhibitor; PTH releaser; a selective androgen receptor molecule (SARM); metalloprotease (MMP) inhibitor; or strontium ranelate.
   Accordingly, in another aspect, the present invention provides:
5. A pharmaceutical combination comprising a) rapamycin or a rapamycin derivative, and b) a second drug, e.g. as exemplified above.
6. A method as defined above comprising co-administration, e.g. concomitantly or in sequence, of a therapeutically effective amount of rapamycin or a rapamycin derivative, and a second drug, e.g. as exemplified above.

The terms "co-administration" or "combined administration" or the like as utilized herein are meant to encompass administration of the selected therapeutic agents to a single patient, and are intended to include treatment regimens in which the drugs are administered together, one after the other or separately in one combined unit dosage form or in two separate unit dosage forms but also in which the drugs are not necessarily administered by the same route of administration or at the same time. A unit dosage form may also be a fixed combination.

Utility of the compounds of the invention in treating diseases and conditions as hereinabove specified, may be demonstrated in standard animal or clinical tests, e.g. in accordance with the methods described hereinafter.

### A. In vitro

### A.1 Mouse Osteoclastogenesis Assay

Non-adherent bone marrow mononuclear cells from 5-week-old male mice cells are differentiated into bone-resorbing osteoclasts by treatment with a cytokine cocktail containing receptor activator of NF kappa B ligand (RANKL), macrophage-colony stimulating factor (M-CSF) and interleukin-1 (IL-1) alpha. Osteoclast formation is measured after 6 days by quantifying the number of tartrate-resistant acid phosphatase (TRAP)-positive multinucleated cells generated in plastic wells on a 48-well plate. Osteoclast activity is measured after 12 days by quantifying the area of resorbed dentine slices placed in wells on a 48-well plate. Treatment with rapamycin or the rapamycin derivative, e.g. Compound A, starts at the beginning of cell culture, together with the cytokine treatment.

Osteoblast differentiation is evaluated in mouse pre-osteoblastic cell line MC3T3-1 b, stimulated to differentiate with osteogenic stimulus (a mixture of bone morphogenetic protein 2 (BMP-2), ascorbic acid and beta-glycerophosphate). Osteoplast activity is measured by quantifying culture area covered with alkaline phosphate-positive cells on a 48-well plate. Treatment with rapamycin or the rapamycin derivative, e.g. Compound A, starts at the beginning of cell culture, together with the osteogenic stimulus treatment.

In this assay, rapamycin or the rapamycin derivatives inhibit osteoclast formation and activity at an IC₅₀ < 1µm.

Using Compound A, osteoclast formation is inhibited with an IC₅₀ of 10.5 ± 4.6 nM and osteoclast activity with an IC₅₀ of 0.6 ± 0.3 nM for osteoclast activity. Alkaline phosphatase (ALP) staining has an IC₅₀ of 13.5 ± 2.4 nM.

### A.2 Human Osteoclastogenesis Assay

Peripheral blood mononuclear cells from healthy male donors are differentiated into bone-resorbing osteoclasts by treatment with a cytokine cocktail containing RANKL, M-CSF and transforming growth factor (TGF)-beta 1. Osteoclast formation is measured after 17 days by quantifying the number of TRAP-positive multinucleated cells generated in plastic wells on a 96-well plate. Osteoclast activity is measured after 17 days by quantifying the area of resorbed bone on bovine cortical bone slices placed in wells on a 96-well plate. Treatment with rapamycin or the rapamycin derivative, e.g. Compound A, starts at the beginning of cell culture, together with the cytokine treatment. Collagen fragments are measured by enzyme linked immunosorbent assay (ELISA).

In this assay, rapamycin or the rapamycin derivatives inhibit osteoclast formation at an IC₅₀ < 1µm.

Using Compound A, osteoclast formation is inhibited with an IC₅₀ values of 7.7 ± 1.1 nM. Resorbed ares is inhibited with an IC₅₀ of 3.4 ± 0.3 nM. Collagen fragment release is inhibited with an IC₅₀ of 4.0 ± 0.5 nM.

Rapamycin and rapamycin derivatives are evaluated for in vivo bone resorption inhibition in an animal model e.g. as disclosed in Shinoda et al., Calcif. Tissue Int., 1983, 35, 87-99 or Schenk et al. Calcif. Tissue Res. 1973, 11, 196-214, or e.g. as disclosed hereinafter.

A.3 Gene expression is analyzed according to a method known in the art, in human osteoclasts after treatment with rapamycin or a derivative thereof. In particular, it is found that the expression of the osteoclast-specific protease cathepsin K is reduced, e.g. by about 78% for Compound A, and the expression of the Cdc2-related serine/threonine PFTAIRE1 is increased, e.g. by about 300% for Compound A.

### B. In vivo: Ovariectomized rat model

Before operation, the tibial bone mass and geometry of the animals is measured at baseline by dual-energy x-ray absorptiometry (DEXA) and periphere quantitative computer tomography (pQCT). Following ovariectomy (OVX) or sham operation, skeletally mature rats are treated for 8 weeks daily with 0.15 mg/kg, 0.5 mg/kg, 1.5 mg/kg, or 3.0 mg/kg of rapamycin or a rapamycin derivative, e.g. Compound A, or vehicle alone by oral administration or once a week with 1.5 mg/kg or 5.0 mg/kg of rapamycin or a rapamycin derivative, e.g. Compound A. At the beginning of the treatment, animals receive a fluorochrome label, e.g. calcein (e.g. 30mg/kg, subcutaneous (s.c.)). Changes in bone mass and geometry (pQCT, DEXA) are evaluated in vivo after 4 weeks of treatment and at 8 weeks before necropsy. Body weight is monitored weekly. The animals are administered two further fluorochrome labels for marking of bone mineralization prior to necropsy, e.g. alizarin e.g. 20mg/kg, s.c., 10 days prior to necropsy, and calcein e.g. 30mg/kg, s.c., 3 days prior to necropsy. Blood samples (500µl blood) are taken in heparin before necropsy and frozen at -20°C for analysis of calcium, phosphate, TRAP, ALP, and osteocalcin. DEXA measurements are carried out at necropsy on excised tibia, femur, and lumbar vertebrae.

For example, Compound A reduces cancellous bone loss with 60% inhibition at 3 mg/kg/day, and inhibits reduction of trabecular number.

Daily dosages required in practicing the method of the present invention when rapamycin or a rapamycin derivative alone is used will vary depending upon, for example, the compound used, the host, the mode of administration and the severity of the condition to be treated. A preferred daily dosage range is about from 0.1 to 25 mg as a single dose or in divided doses. Suitable daily dosages for patients are on the order of from e.g. 0.1 to 25 mg p.o. Rapamycin or a rapamycin derivative may be administered by any conventional route, in particular enterally, e.g. orally, e.g. in the form of tablets, capsules, drink solutions, nasally, pulmonary (by inhalation) or parenterally, e.g. in the form of injectable solutions or suspensions. Suitable unit dosage forms for oral administration comprise from ca. 0.05 to 12.5 mg, usually 0.25 to 10 mg of rapamycin or a rapamycin derivative, e.g. Compound A, together with one or more pharmaceutically acceptable diluents or carriers therefor.

Due to synergism lower doses of the drugs of the combination of the invention may be used, for example, the dosages need not only often be smaller but are also applied less frequently, or may be used in order to diminish the incidence of side-effects. This is in accordance with the desires and requirements of the patients to be treated.

When the rapamycin derivative is co-administered with a second drug, dosages for the co-administered drug will of course vary depending on the type of drug employed, e.g. whether it is a steroid, a calcitonin or a biphosphonate, on the specific drug employed, on the condition to be treated, the severity of the condition being treated, whether it is a curative or preventive therapy, on the regimen and so forth.

The pharmaceutical compositions for separate administration of a rapamycin derivative and a second drug and for the administration in a fixed combination, i.e. a single galenical composition comprising at least two combination partners, according to the invention may be prepared in a manner known per se comprising a therapeutically effective amount of at least one pharmacologically active combination partner alone, e.g. as indicated above, or in combination with one or more pharmaceutically acceptable carriers or diluents.

## Claims

1. Use of a rapamycine derivative of formula 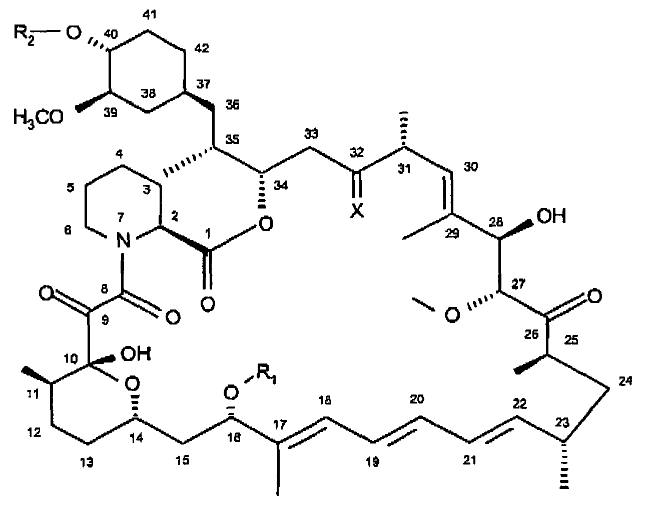 wherein
R₁ is CH₃ or C₃₋₆alkynyl,
R₂ is H or -CH₂-CH₂-OH, 3-hydroxy-2- (hydroxymethyl)-2-methyl-propanoyl, or tetrazolyl, and
X is =O, (H, H) or (H, OH),
provided that R₂ is other than H when X is=O and R₁ is CH₃,
or a physiologically hydrolysable ether thereof when R₂ is-CH₂-CH₂-OH, in the preparation of a pharmaceutical composition for the treatment of abnormally increased bone turnover or resorption.

2. A pharmaceutical composition for use in the treatment of abnormally increased bone turnover or resorption, comprising a rapamycin derivative of formula 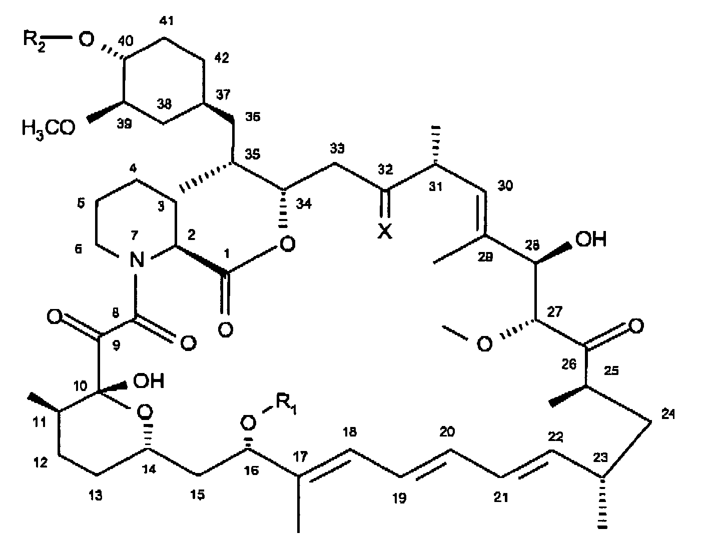 wherein
R₁ is CH₃ or C₃₋₆alkynyl,
R₂ is H or -CH₂-CH₂OH, 3-hydroxy-2- (hydroxymethyl)-2-methyl-propanoyl, or tetrazolyl, and
X is =O, (H, H) or (H, OH),
provided that R₂ is other than H when X is=O and R₁ is CH₃,
or a physiologically hydrolysable ether thereof when R₂ is-CH₂-CH₂-OH,
together with one or morepharmaceutically acceptable diluents or carriers therefor.

3. A pharmaceutical combination comprising a rapamycin derivative of formula 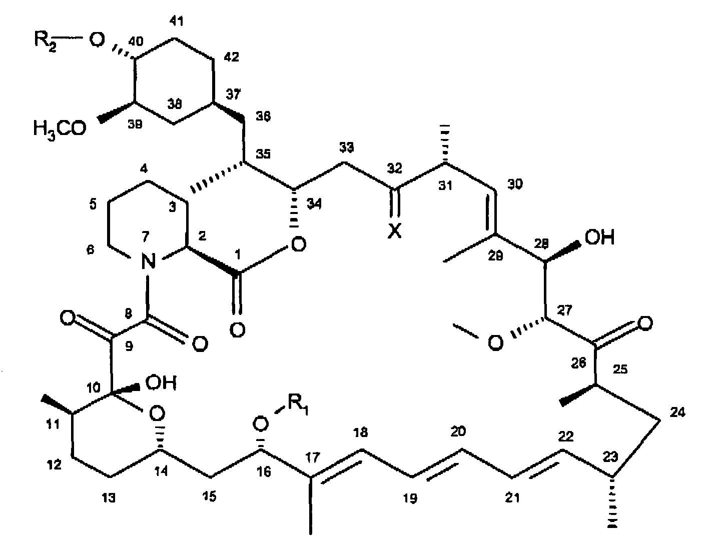 wherein
R₁ is CH₃ or C₃₋₆alkynyl,
R₂ is H or -CH₂-CH₂-OH, 3-hydroxy-2-(hydroxymethyl)-2-methyl-propanoyl, or tetrazolyl, and
X is =O, (H, H) or (H, OH),
provided that R₂ is other than H when X is=O and R₁ is CH₃,
or a physiologically hydrolysable ether thereof when R₂ is-CH₂-CH₂-OH,
and a second drug selected from
a calcitonin;
a steroid hormone,
Parathyroid Hormone (PTH), PTH(1-84), PTH (1-34), PTH (1-36), PTH(1-38), PTH (1-31)NH₂ or PTS893,
a bisphosphonate;
a cathepsin K inhibitor;
a selective androgen receptor modulator ; and
strontium ranelate.

4. A pharmaceutical combination according to claim 3, wherein the second drug is selected from the group consisting of salmon, eel or human calcitonin; raloxifene, lasofoxifene, TSE-424, FC1271; tibolone (Livial®), Parathyroid Hormone (PTH), PTH(1-84), PTH (1-34), PTH (1-36), PTH(1-38), PTH (1-31)NH₂, PTS893 ; alendronate, zoledronic acid, ibandronate and strontium ranelate.

5. Use, composition or combination according to any preceding claim wherein the rapamycin derivative is selected from40-0- (2-hydroxyethyl)rapamycin, 40- [3- hydroxy-2- (hydroxymethyl)-2-methylpropanoate]-rapamycin, 40-epi- (tetrazolyl)-rapamycin, 32-deoxorapamycin, 16-pent-2-ynyloxy-32 (S) -dihydro rapamycin, and TAFA-93.

6. Use, composition or combination according to any preceding claim wherein the rapamycin derivative is 40-O-(2-hydroxyethyl)rapamycin.

7. Use, composition or combination according to any preceding claim for the treatment of osteoporosis; bone loss secondary to or due to medication; bone loss associated with immobilisation and space flight ; bone loss associated with rheumatoid arthritis, osteopenia, osteogenesis imperfecta, hyperthyroidism, anorexia nervosa, organ transplantation, joint prosthesis loosening ;periarticular bone erosions in rheumatoid arthritis; osteoarthritis; hypercalcemia ; bone cancer and bone metastases;and/or multiple myeloma.

## Patentansprüche

1. Verwendung eines Rapamycinderivats der Formel 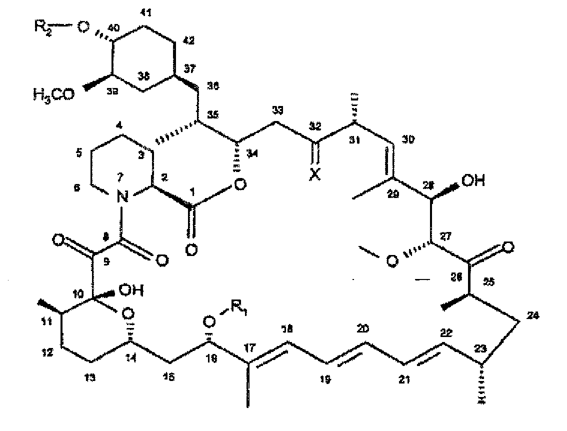 worin
R₁ für CH₃ oder C₃-C₆-Alkinyl steht,
R₂ für H oder -CH₂-CH₂-OH, 3-Hydroxy-2-(hydroxymethyl)-2-methylpropanoyl oder Tetrazolyl steht, und
X für =O, (H, H) oder (H, OH) steht,
mit der Maßgabe, dass R₂ etwas anderes als H ist, wenn X für =O steht und R₁ für CH₃ steht,
oder ein physiologisch hydrolysierbarer Ether hiervon, wenn R₂ für -CH₂-CH₂-OH steht,
zur Herstellung einer pharmazeutischen Zusammensetzung für die Behandlung einer abnormal erhöhten Knochenumwandlung oder Knochenresorption.

2. Pharmazeutische Zusammensetzung zur Verwendung für die Behandlung einer abnormal erhöhten Knochenumwandiung oder Knochenresorption, umfassend ein Rapamycinderivat der Formel 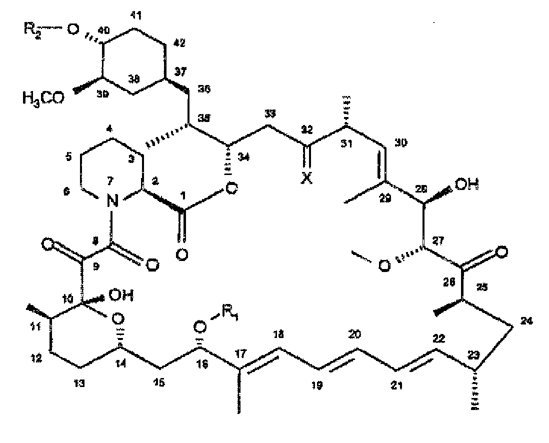 worin
R₁ für CH₃ oder C₃-C₆-Alkinyl steht,
R₂ für H oder -CH₂-CH₂-OH, 3-Hydroxy-2-(hydroxymethyl)-2-methylpropanoyl oder Tetrazolyl steht, und
X für =O, (H, H) oder (H, OH) steht,
mit der Maßgabe, dass R₂ etwas anderes als H ist, wenn X für =O steht und R₁ für CH₃ steht,
oder ein physiologisch hydrolysierbarer Ether hiervon, wenn R₂ für -CH₂-CH₂-OH steht,
zusammen mit ein oder mehr pharmazeutisch akzeptablen Verdünnungsmitteln oder Trägern hierfür.

3. Pharmazeutische Kombination, umfassend ein Rapamycinderivat der Formel 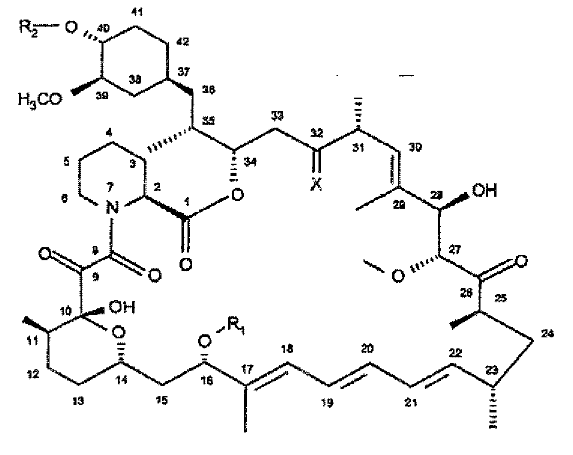 worin
R₁ für CH₃ oder C₃-C₆-Alkinyl steht,
R₂ für H oder -CH₂-CH₂-OH, 3-Hydroxy-2-(hydroxymethyl)-2-methylpropanoyl oder Tetrazolyl steht, und
X für =O, (H, H) oder (H, OH) steht,
mit der Maßgabe, dass R₂ etwas anderes als H ist, wenn X für=O steht und R₁ für CH₃ steht,
oder ein physiologisch hydrolysierbarer Ether hiervon, wenn R₂ für -CH₂-CH₂-OH steht,
und einen zweiten Wirkstoff, der ausgewählt aus einem
Calcitonin,
Steroidhormon,
Parathormon (PTH), PTH (1 -84), PTH (1-34), PTH (1-36), PTH (1-38). PTH (1-31)NH₂ oder PTS 893,
Bisphosphonat,
Kathepsin-K Inhibitor,
selektiven Androgenrezeptormodulator, und
Strontiumranelat.

4. Pharmazeutische Kombination nach Anspruch 3, worin der zweite Wirkstoff ausgewählt ist aus der Gruppe, die besteht aus Salm-, Aal- oder Humancalcitonin, Raloxifen, Lasofoxifen, TSE-424, FC 1271, Tibolon (Livial ^{®}), Parathormon (PTH), PTH (1-84), PTH (1-34), PTH (1-36), PTH (1-38), PTH (1-31)NH_{2,} PTS 893, Alendronat, Zoledronsäure, Ibandronat und Strontiumranelat.

5. Verwendung einer Zusammensetzung oder Kombination nach einem der vorhergehenden Ansprüche, worin das Rapamycinderivat ausgewählt ist aus 40-O-(2-Hydroxyethyl)-rapamycin, 40-[3-Hydroxy-2-(Hydroxymethyl)-2-methylpropanoat]-rapamycin, 40-Epi-(tetrazolyl)-rapamycin, 32-Deoxorapamycin, 16-Pent-2-inyloxy-32(S)-dihydrorapamycin und TAFA-93.

6. Verwendung einer Zusammensetzung oder Kombination nach einem der vorhergehenden Ansprüche, worin das Rapamycinderivat 40-O-(2-Hydroxyethyl)-rapamycin ist.

7. Verwendung einer Zusammensetzung oder Kombination nach einem der vorhergehenden Ansprüche zur Behandlung von Osteoporose, Knochenverlust sekundär zu oder infolge einer Medikation, Knochenverlust in Assoziation mit einer Immobilisation und einem Raumverlust, Knochenverlust in Assoziation mit rheumatoider Arthritis, Osteopenie, Osteogenese imperfecta, Hyperthyroidismus, Anorexia nervosa, Organtransplantation, Lockerung der Verbindungsstelle einer Prothese, periartikulare Knochenerosionen bei rheumatoider Arthritis, Osteoarthritis, Hypercalcämie, Knochenkrebs und Knochenmetastasen und/oder multiplem Myelom.

## Revendications

1. Utilisation d'un dérivé de rapamycine de formula : 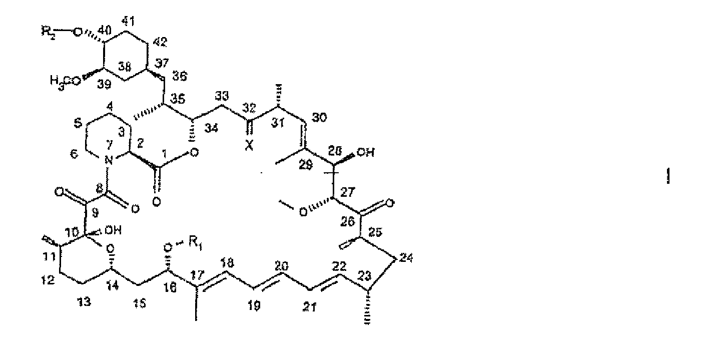 dans laquelle
R₁ représente un groupe CH₃ ou alcynyle en C₃₋₆,
R₂ représente H ou un groupe -CH₂-CH₂-OH, 3-hydroxy-2-(hydroxyméthyl)-2-méthyl-propanoyle ou tétrazolyle,
et
X est =O, (H, H) ou (H, OH)
à la condition que R₂ ne soit pas H lorsque X est =O et R₁ est CH₃,
ou un éther physiologiquement hydrolysable de celui-ci lorsque R₂ est -CH₂-CH₂-OH, dans la préparation d'une composition pharmaceutique destinée au traitement du renouvellement osseux ou de la résorption osseuse anormalement accrûs.

2. Composition pharmaceutique à utiliser dans le traitement du renouvellement osseux ou de la résorption osseuse anormalement accrus, comprenant un dérivé de rapamycine de formule 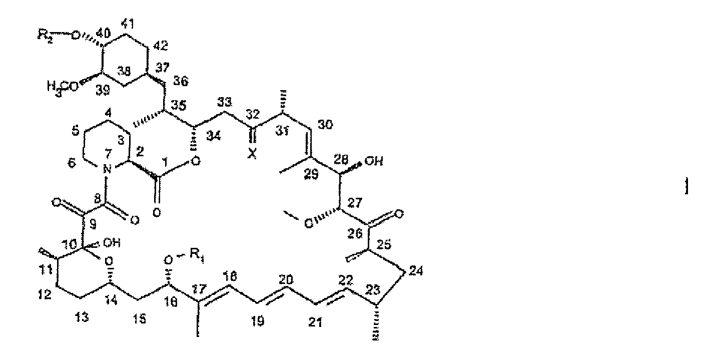 dans laquelle
R₁ représente un groupe CH₃ ou alcynyle en C₃₋₆,
R₂ représente H ou un groupe -CH₂-CH₂-OH, 3-hydroxy-2-(hydroxymëthyl)-2-mëthyl-propanoyle ou tétrazolyle,
et
X est =O, (H, H) ou (H, OH)
à la condition que R₂ ne soit pas H lorsque X est =O et R₁ est CH₃,
ou un éther physiologiquement hydrolysable de celui-ci lorsque R₂ est -CH₂-CH₂-OH,
avec un ou plusieurs diluants ou supports de celui-ci acceptables sur le plan pharmaceutique.

3. Combinaison pharmaceutique comprenant un dérivé de rapamycine de formule 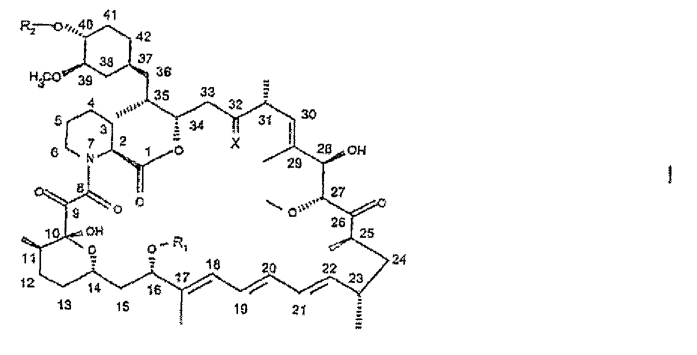 dans laquelle
R₁ représente un groupe CH₃ ou alcynyle en C₃₋₆,
R₂ représente H ou un groupe -CH₂-CH₂-OH, 3-hydroxy-2-(hydroxyméthyl)-2-méthyl-propanoyle ou tétrazolyle,
et
X est =O, (H, H) ou (H, OH)
à la condition que R₂ ne soit pas H lorsque X est =O et R₁ est CH₃,
ou un éther physiologiquement hydrolysable de celui-ci lorsque R₂ est -CH₂-CH₂-OH,
et un second médicament sélectionné parmi
une calcitonine ;
une hormone stéroïde,
une hormone parathyroïdienne (PTH), PTH(1-84), PTH (1-34), PTH (1-36), PTH (1-38), PTH (1-31)-NH₂ ou PTS 893 ;
un bisphosphonate ;
un inhibiteur de cathepsine K ;
un modulateur sélectif du récepteur des androgènes ; et
le ranélate de strontium.

4. Combinaison pharmaceutique selon la revendication 3, dans laquelle le second médicament est sélectionné dans le groupe composé de la calcitonine de saumon, d'anguille ou d'humain ; du raloxifène, du lasofoxifène, du TSE-424, du FC1271 ; de la tibolone (Livial®) ; de l'hormone parathyroïde (PTH), de PTH (1-84), PTH (1-34), PTH (1-36), PTH (1-38), PTH (1-31)-NH₂ ou de PTS 893 ; de l'alendronate, de l'acide zolédronique, de l'ibandronate et du ranélate de strontium.

5. Utilisation, composition ou combinaison selon l'une quelconque des revendications précédentes, dans laquelle le dérivé de rapamycine est sélectionné parmi la 40-O-(2-hydroxyéthyl)-rapamycine, la 40-[3-hydroxy-2-(hydroxyméthyl)-2-méthylpropanoate]-rapamycine, la 40-épi-(tétrazolyl)rapamycine, la 32-désoxorapamycine, la 16-pent-2-ynyloxy-32(S)-dihydro-rapamycine, ou TAFA-93.

6. Utilisation, composition ou combinaison selon l'une quelconque des revendications précédentes, dans laquelle le dérivé de rapamycine est la 40-O-(2-hydroxyéthyl)-rapamycine.

7. Utilisation, composition ou combinaison selon l'une quelconque des revendications précédentes pour traiter l'ostéoporose; la perte osseuse secondaire à ou due à un médicament ; la perte osseuse associée à une immobilisation et à la navigation spatiale ; la perte osseuse associée à la polyarthrite rhumatoïde, l'ostéopénie, l'ostéogenèse imparfaite, l'hyperthyroïdie, l'anorexie mentale, une transplantation d'organe, un descellement de prothèse articulaire ; les érosions osseuses périarticulaires dans la polyarthrite rhumatoïde ; l'ostéo-arthrite ; l'hypercalcémie ; le cancer osseux et les métastases osseuses ; et/ou un myélome multiple.
